Europäisches Patentamt

European Patent Office

Office européen des brevets

(11) Publication number: **0 125 818**
A1

# EUROPEAN PATENT APPLICATION

(21) Application number: **84302700.4**

(22) Date of filing: **19.04.84**

(51) Int. Cl.³: **C 12 N 15/00**
C 12 P 21/00, C 12 P 19/34
//C12R1/19

(30) Priority: **19.04.83 US 486505**

(43) Date of publication of application:
**21.11.84 Bulletin 84/47**

(84) Designated Contracting States:
**AT BE CH DE FR GB IT LI LU NL SE**

(71) Applicant: GENEX CORPORATION
6110 Executive Boulevard
Rockville Maryland 20852(US)

(72) Inventor: Vaslet, Charles A.
10118 Crestwood Road
Kensington Maryland(US)

(74) Representative: Holmes, Michael John et al,
Frank B. Dehn & Co. Imperial House 15-19 Kingsway
London WC2B 6UZ(GB)

(54) Cloned ovine growth hormone gene.

(57) There is disclosed a cloned ovine growth hormone gene, which may be comprised in a cloned ovine pregrowth hormone gene, and microorganisms transformed with plasmids comprising such genes. Techniques for the production of such genes, plasmids and micro-organisms are also disclosed.

EP 0 125 818 A1

1

The present invention relates to a cloned ovine gene, which specifies the biosynthesis of ovine growth hormone (OGH). The invention further relates to a plasmid containing a cloned ovine gene as well as a microorganism transformed with such a plasmid.

Animal growth hormones are members of a family of anabolic proteins which promote tissue growth and regulate other phases of protein metabolism as well as fat, carbohydrate, and mineral metabolism. In general, growth hormones are single chain, globular proteins with molecular weights of 22,000 to 24,000 daltons. The mature hormone is about 190 to 200 amino acids in length. See Barrington, E.J., Comparative Biochemistry of Growth Hormones, Prolactin, and Glycoprotein Hormones; Hormones and Evolution, Academic Press, N.Y., Pub. Vol. 2 (1979). Such hormones are produced in vertebrates by the anterior lobe of the pituitary gland, otherwise known as the adenohypophysis.

It is generally known that growth hormones are species-specific. In addition to disparities in biological responses, growth hormones from different species vary in their isoelectric points, N-terminal and C-terminal amino acid residues and amino acid composition.

Complete amino acid sequence information is available for human, bovine, ovine, equine, and rat growth hormones. See Dayhoff, M.O. (1976), Atlas of Protein Sequences and Structure, National Biomedical Research Foundation, publ. Vol. 5: suppl. 2. All of these growth hormones appear to be highly conserved proteins with long stretches of interspecies sequence homology in their primary amino acid sequences. When naturally synthesized in the cell, these hormones are extended at their N-termini by "signal" or "pre" sequences, which effect the passage of newly

2

synthesized hormone through the membrane of the endoplasmic reticulum. The "pre" peptide is eventually cleaved to produce the mature hormone. See, Davies, B.D. and Tai, P.C., The Mechanism of Protein Secretion Across Membranes, Nature 283:433-438 (1980).

Growth hormone is of particular interest to the livestock industry, as growth hormone is required for the normal, balanced growth of pre-adult animals. Growth-promoting effects are due primarily to an ability to increase the retention of nitrogen as protein in the tissues by increasing membrane transport of amino acids into cells and stimulating translation of RNA into protein at the ribosomal level. Growth hormone also causes an increase in mitotic activity and cell division, two essential conditions for growth. Animals whose diets are supplemented with growth hormone gain additional weight as a result of increased tissue protein and salt production. Consequently, growth hormone can be used as a means for increasing meat production without significantly increasing the caloric intake of livestock.

Current methods for isolating growth hormone from the pituitary are impractical for producing growth hormone in commercial quantitities. Consequently, efforts have been made to produce growth hormone in commercial quantities at reasonable cost by methods which utilize recombinant deoxyribonucleic acid (DNA) technology. Animal growth hormones from several species have been cloned and expressed in bacterial cells, including those of the human, bovine, and rat. See, e.g., Miller, W.L. et al., Molecular Cloning of DNA Complementary to Bovine Growth mRNA Journal of Biological Chemistry 255:7521-7524; Martial, J.A. et al., Human Growth Hormone: Complementary DNA Cloning and Expression in Bacteria, Science, 205: 602-607, (1979); and Seeburg, P.H. et al., Nucleotide Sequence and Amplication in Bacteria of Structural Gene for Rat Growth Hormone, Nature, 270: 486-494 (1977).

The present invention relates to the gene for ovine growth hormone (sometimes abbreviated here to OGH). Since growth hormones are species-specific, ovine growth hormone is the only growth hormone useful for increasing the rate of meat production or feed efficiency in sheep: There is, therefore, a need to obtain ovine growth hormone in large amounts. According to the invention there is provided a cloned ovine growth hormone gene, which may be comprised in a cloned ovine pre-growth hormone gene and microorganisms transformed with plasmids comprising such genes via conventional techniques of molecular biology. e.g. Ullrich, A., et al., Science, 196, 1313 (1977) and Seeburg, P.H., et al., Nature, 270, 486 (1977) and the cultivation of the microorganism in conditions to yield ovine growth hormone or ovine pre-growth hormone as required. Also provided is an intron-free cloned ovine growth hormone gene and such a gene comprising a cloned pre-ovine growth hormone gene.

The procedures leading to an ovine pre-growth hormone-producing microorganism can be divided into the following major stages, each of which is described more fully herein: (1) Recovery and isolation of ovine pre-growth hormone messenger RNA (mRNA) from ovine pituitary (2) In vitro synthesis of complementary DNA (cDNA), using ovine pre-growth hormone mRNA as a template, (3) Insertion of the cDNA into a suitable cloning vector and transformation of bacterial cells with that cloning vector, and (4) Recovery and isolation of the cloned gene.

Although genes which specify ovine pre-growth hormone are present in the chromosomes of every ovine cell, for reasons discussed below, genes associated with ovine pre-growth hormone are most readily obtained from the ovine pituitary.

Eukaryotic genes are contained in the chromosomal DNA of the cell nuclei. This chromosomal DNA exists in a compact nucleoprotein complex called chromatin. Freeing the chromsomal DNA from the chromatin, and isolating a single

4

gene therefrom has not been a feasible means for obtaining such a gene. Moreover, unlike prokaryotic genes, most eukaryotic genes do not occur as continuous structural sequences, but comprise discontinuous subsequences, which are separated by noninformational segments (introns) of DNA. Prokaryotic transcription and translation of such genes might produce a hybrid protein quite dissimilar from the desired ovine pre-growth hormone, but transcription of such genes and processing of the RNA transcript in the eukaryotic cell result in a messenger RNA free of the noninformational segments. Therefore, the mRNA usually provides the desired genetic information in its most accessible form, i.e., that which is derived from cells which actually synthesize growth hormone.

Anterior pituitary cells are obtained from adult ovine pituitary glands. The ovine growth hormone mRNA produced by the anterior pituitary cells is complementary to the ovine growth hormone gene, and may be employed as a template for the synthesis of complementary DNA (cDNA) as hereinafter described. To effectively utilize the mRNA for the synthesis of cDNA, it is advantageously recovered from the cells in relatively pure form. This recovery involves separating the mRNA not only from the cell membranes, proteins, lipids, carbohydrates, salts, and such, present in the cells, but also from mRNA molecules associated with the biosynthesis of proteins other than the desired ovine growth hormone. The procedures described by Chirgwin, J.M., et al., Biochemistry, 18, 5294-5299 (1979) and Palmeter, R. Biochemistry, 13, 3603-3615 (1974), may be used advantageously for the recovery of ovine growth hormone mRNA. RNA is inherently less stable than DNA, and is particularly subject to degradation by ribonucleases that are present in relatively high concentrations in pituitary cells. Therefore, the mRNA recovery procedures generally employ means for rapidly inactivating any ribonucleases which are present.

In general, recovery of total RNA, is initiated by disrupting the cells in the presence of a ribonuclease-inactivating substance. Disruption of the cells may be accomplished by subjecting the cells to a lysing reagent, freezing/thawing, or mechanical disruption; preferably a combination thereof. Heparin has been found to inhibit ribonuclease activity.

After disruption of the cells, the solids are removed, e.g., by low speed centrifugation. The RNA is preferably recovered from the resulting clarified solution as poly-somal RNA. Polysomal RNA is RNA to which ribsomes have bound, and which, due to translation of the RNA, also contain segments of the growth hormone protein of varying lengths. Recovery of the polysomal RNA can be accomplished by centrifuging the clarified solution through a sucrose gradient (e.g., a 52% sucrose solution) at high speed. The polysomal RNA can be recovered as a pellet at the bottom of the centrifuge tube. The polysomal RNA is then suspended in a buffer, and protein is extracted with a mixture of phenol and chloroform. Salt is added to the aqueous phase of the extract and the polysomal RNA is precipitated with alcohol at low temperature (e.g., -20°C to -80°C).

Affinity chromatography can be used to further purify ovine growth hormone mRNA. Since OGH mRNA is polyadenylated at the 3' end, it can readily be separated from non-polyadenylated RNA by oligo (dT)-cellulose affinity chromatography (Green, M., et al., Arch. Biochem. Biophys., 172, 74 [1976]). Polyadenylated RNA generally makes up only about 1-4% of total RNA. Further enrichment for OGH mRNA can be achieved by fractionation on continous sucrose density gradients.

Cell-free translation of polyadenylated mRNA (and intermediate fractions if desired) can be employed to confirm the integrity of the final mRNA preparation and its degree of enrichment in OGH mRNA.  A number of cell-free translational systems have been devised, such as wheat germ extract (Martial, J., et al., Proc. Nat. Acad. Sci. USA, 74, 1816 [1977]), and mRNA-dependent riticulocyte lysate (Pelham, H.R.B., et al., Eur. J. Biochem., 67, 247 [1976]). Translation of the recovered and isolated OGH mRNA is preferably conducted in a rabbit reticulocyte system (available from Bethesda Research Laboratories of Rockville, MD), which is supplemented with radioactively labelled methionine ($^{35}$S-methionine).  The products of this translation can be visualized by sodium dodecyl sulfate-polyacrylamide gel electrophoresis and fluorography.  The mRNA fractions having the highest activity by the rabbit reticulocyte lysate test can be combined for use in cDNA synthesis.

The preparation of cDNA involves enzymatically constructing double-stranded DNA, which has a nucleotide base pair sequence identical to the functional sequence of the original chromosomal gene.  As indicated above, the cDNA does not contain the noninformational segments of the eukaryotic gene, and thus can ultimately be transcribed and translated in prokaryotic systems.

Synthesis of cDNA employs avian myeloblastosis virus reverse transcriptase.  This enzyme catalyzes the synthesis of a single-stranded copy from deoxynucleoside triphosphates on the mRNA template.  Kacien, D.L., et al., Proc. Nat'l Acad. Sci. USA, 73, 2191 (1976).  The poly (rA) tail of mRNA permits oligo (dT) (of about 12-18 nucleotides) to be used as a primer for cDNA synthesis. The use of a radioactively-labelled deoxynucleoside triphosphate facilitates monitoring of the synthesis reaction.  Generally, a $^{32}$P-containing deoxynucleoside triphosphate, such as [$\alpha$-$^{32}$P]dCTP may be used for this purpose.  The cDNA synthesis is generally conducted by combining the mRNA, the deoxynucleoside triphosphates, the oligo (dT) and the reverse transcriptase in a properly buffered solution. The solution also preferably contains small amounts of actinomycin D and dithiothreitol to promote full length synthesis. Kacien, D.L., et al. supra. This solution is incubated at an elevated temperature, e.g, about 40-50°C, for a time sufficient to allow formation of the cDNA copy, e.g. about 5-20 minutes. After incubation, ethylenediaminetetraacetic acid is added to the solution, and the solution extracted with phenol:chloroform (1:1 by vol.). The aqueous phase is advantageously purified by gel filtration chromatography, and the cDNA-mRNA complex in the eluate precipitated with alcohol.

The mRNA, being inherently less stable than the cDNA, can be hydrolyzed with an alkaline solution such as dilute sodium hydroxide (about 0.1 M) at an elevated temperature, e.g., about 60-80°C for about 15-30 minutes. Neutralization of the alkaline solution and alcohol precipitation yields a single-stranded cDNA copy.

The single-stranded cDNA copy has been shown to have a 5'-poly (dT) tail, and to have a 3' terminal hairpin structure, which provides a short segment of duplex DNA. Efstratiadis, A., et al., Cell, 7, 279 (1976). This 3' hairpin structure can act as a primer for the synthesis of a complementary DNA strand. Synthesis of this complementary strand is conducted under essentially the same conditions as the synthesis of the cDNA copy, except that the Klenow fragment of DNA polymerase I (Klenow, H., et al., Eur. J. Biochem., 22, 371 [1977]) is substituted for reverse transcriptase. The duplex cDNA recovered by this procedure has a 3' loop, resulting from the 3' hairpin structure of the single-stranded cDNA copy. This 3' loop can be cleaved by digestion with the enzyme, S1 nuclease, using essentially the procedure of Ullrich, A., et al., supra. The S1 nuclease digest may be extracted with phenol-chloroform, and the resulting cDNA precipitated from the aqueous phase with alcohol.

The double-stranded cDNA obtained by the foregoing procedures is advantageously amplified in vitro by inserting it into a suitable cloning vector, and transforming an appropriate host cell with the modified cloning vector. Suitable cloning vectors include various plasmids and phages, and plasmids are generally preferred for such amplification procedures. The criteria for selecting a cloning vector include its size, its capability for replicating in the host cells, the presence of selectable genes, and the presence of a site for the insertion of the gene. With respect to its size, the vector is advantageously relatively small, to permit large gene insertions, and so as not to divert large amounts of cellular nutrients and energy to the production of unwanted macromolecules. The vector also includes an intact replicon which remains functional after insertion of the gene. This replicon preferably directs the desired mode of replication of the plasmid, i.e., multiple copies or a single copy per cell, or a controllable number of copies per cell. Genes specifying one or more phenotypic properties, preferably antibiotic resistance, facilitate selection of transformants. The insertion site is advantageously a unique restriction site for a restriction endonuclease. A cloning vector meeting all of these criteria is the plasmid pBR322. Bolivar, F., et al., Gene, 2, 95 (1977). This plasmid is small (about $2.8 \times 10^6$ daltons), carries genes for ampicillin (amp) and tetracycline (tet) resistance, and is subject to relaxed replication in E.coli. The plasmid also has a single restriction site for the endonuclease, PstI, which occurs within the amp gene. The cDNA can be conveniently inserted into this plasmid by a homopolymeric tailing technique. Nelson, T., et al., Methods of Enzymology, 68, 41 (1980). Homopolymer tails, e.g., poly-dC, are added to the 3'-hydroxyls of the ovine pregrowth hormone cDNA, by reaction with the appropriate deoxynucleoside triphosphate, e.g., dCTP, in the presence of terminal deoxynucleotidyl transferase (Chang, L.M.S., et al., J. Biol. Chem., 246, 909 [1971]). The resulting full-length poly-dC-tailed cDNA molecules are sized on neutral sucrose gradients by the procedure of Norgard et al., J. Biol. Chem., 255, 7665-7672 (1980). The plasmid is opened by digestion with the appropriate endonuclease, such as Pst I, and complementary homopolymer tails, e.g., poly dG, are added to the 3'-hydroxyls of the opened plasmid, using the identical homopolymer tailing technique e.g., using dGTP. If desired, the tailing reactions may be monitored, by employing radioactively labelled deoxynucleotide triphospates e.g., $[^3H]$dCTP and $[^3H]$dGTP, in the reactions. Generally, the reactions are conducted to provide tails of about 10-20 nucleotides long. The tailed cDNA and plasmid are recovered, e.g., by phenol extraction followed by alcohol precipitation. The two "tailed" DNA species are annealed by incubating a buffered solution of the two species to yield a recombinant plasmid containing the ovine pre-growth hormone gene.

A suitable amp$^S$, tet$^S$ strain of E.coli (e.g., strain HB101) may be transformed with the recombinant plasmid, using essentially the method of Lederberg, J. Bacteriology, 119, 1072 (1974). Transformants are typically grown on a standard L-broth, containing tetracycline. Samples of colonies growing on the tetracycline-containing medium are then transferred to a second medium containing amplicillin. Because the pBR322 plasmid imparts tetracycline resistance to the cells, colonies growing on the tetracycline-containing medium must contain that plasmid. On the other hand, the PstI site occurs within the ampicillin resistance gene of the pBR322 plasmid site, therefore, ampicillin resistance is destroyed by insertion of the gene therein. Thus, only tet$^r$, amp$^S$ colonies are selected for further analysis.

10

Generally, several hundred to several thousand potential ovine growth hormone clones are produced by these procedures. A modification of the Grunstein-Hogness in situ hybridization method is employed to screen $amp^s$-$tet^r$ bacterial transformants for OGH-containing sequences. See, Grunstein, M., et al., Proc. Nat'l. Acad. Sci. USA, 72, 3961-3965 (1975). To identify those colonies which contain the OGH gene by the Grunstein-Hogness procedure, a radioactively labeled DNA probe is employed. Four different probes which can be used to positively select the OGH gene-containing recombinants include, in order of preference, (1) $P^{32}$-labelled animal growth hormone DNA from another species, (2) $P^{32}$-labelled cDNA prepared from purified OGH mRNA; (3) $P^{32}$-labelled purified OGH mRNA; and (4) a $P^{32}$-labelled synthetic DNA probe which shares sequence homology with the OGH gene.

To screen the OGH clone candidates, DNA from each colony (or from groups of colonies) is fixed to discrete zones of a nitrocellulose filter and denatured. A solution of the probe is applied thereto under hybridizing conditions. Unhybridized probe is washed from the filter, and colonies containing DNA to which the probe hybridized are identified by autoradiography.

Positive clones may be grown on suitable growth media to obtain ample quantities of cells from which to extract the plasmid DNA. The plasmid DNA is extracted, using conventional techniques, such as disruption of the cells, followed by phenol extraction, and alcohol precipitation. The DNA may be separated, e.g. by electrophoresis on 1% agarose gels or sucrose gradient sedimentation to estimate the length of their inserted sequence. See Klein, R.D. et al., Volume 3, Plasmid, 88-91, (1980). Plasmid DNA containing inserts larger than about 500 base pairs is selected for restriction mapping and DNA sequencing.

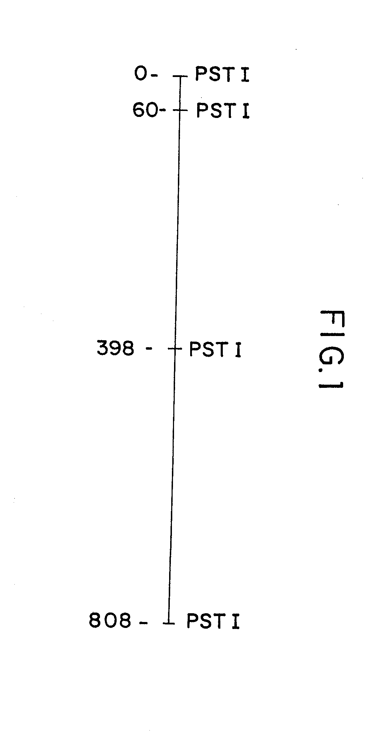

A cell culture containing the cloned pre-OGH gene, which was produced by these procedures, has been deposited with the U.S. Department of Agriculture, Northern Regional Research Laboratories, Peoria, Illinois, U.S.A., as NRRL No. B-15060. A partial restriction map of the cloned pre-OGH gene-containing insert is shown in Figure 1 of the drawings. The insert consists of 808 base pairs, and has noncoding regions at the 5' and 3' ends. The pre-OGH gene is specified by the region from base pair 49 through base pair 699 and the mature OGH gene is specified by the region from base pair 127 through base pair 699.

The nucleotide sequence of the insert can be determined by the method of Sanger, et al., Proc. Nat'l. Acad. Sci. USA, 74, 5463-5467 (1977) or the method of Maxam, et al., Proc. Nat'l. Acad. Sci. USA, 74, 560-564 (1977). This nucleotide sequence is shown in Fig. 2 of the drawings. The drawings show the 5'-->3' strands of the noncoding and coding regions, along with the amino acid sequence specified by the gene. As used in Figure 2 and elsewhere herein, the abbreviations have the following standard meanings:

| | | |
|---|---|---|
| A | = | deoxyadenyl |
| T | = | thymidyl |
| G | = | deoxyguanyl |
| C | = | deoxycytosyl |
| GLY | = | glycine |
| ALA | = | alanine |
| VAL | = | valine |
| LEU | = | leucine |
| ILE | = | isoleucine |
| SER | = | serine |
| THR | = | threonine |
| PHE | = | phenylalanine |
| TYR | = | tyrosine |
| TRP | = | tryptophan |
| CYS | = | cysteine |
| MET | = | methionine |
| ASP | = | aspartic acid |
| GLU | = | glutamic acid |
| LYS | = | lysine |
| ARG | = | arginine |
| HIS | = | histidine |
| PRO | = | proline |
| GLN | = | glutamine |
| ASN | = | asparagine |

It will be appreciated that because of the degeneracy of the genetic code, the nucleotide sequence of the gene can vary substantially. For example, portions or all of the gene could be chemically synthesized to yield DNA having a different nucleotide sequence than that shown in Figure 2, yet the amino acid sequence would be preserved, provided that the proper codon-amino acid assignments were observed. Having established the nucleotide sequence of the pre-OGH gene and the amino acid sequence of the protein, the gene of the present invention is not limited to a particular nucleotide sequence, but includes all variations thereof as permitted by the genetic code.

The present invention has been described in connection with the use of E.coli as the bacterial host for recombinant DNA containing the OGH gene, but skilled molecular biologists will appreciate that other gram-negative bacteria, such as Pseudomonas; gram-positive bacteria, such as Bacillus; and higher unicellular organisms, such as yeasts and fungi can be employed for cloning and/or expression of the OGH gene or portions thereof.

The invention is further illustrated by reference to the following examples, which are not intended to be limiting.

## Example I
### Preparation of Polysomal RNA from Ovine Pituitary

Fresh ovine pituitary glands (mixed sex) were obtained within 1 hour after the animals were sacrificed and frozen immediately in liquid nitrogen. Polysomal RNA was prepared according to a modification of the procedure found in Vaslet et al., Nature 285:674-676 (1980). Several grams of anterior pituitary tissue were homogenized on ice in homogenization buffer comprising 0.25M KCl, 0.025 M $MgCl_2$, 0.05M tris-HCl pH 7.4, 0.5% NP40, 200 µg/ml cycloheximide and 250 µg/ml heparin. The tissue was first minced with scissors and then homogenized in a Virtis 45 homogenizer on setting 70 for 5 minutes. The homogenate was transferred to 15 ml Corex® tubes and centrifuged at 15,000 RPM for 30 minutes at 4°C. Polysomes were collected as a pellet prepared by centrifuging the low speed supernatant through a 2.5 mil cushion of fifty-two percent sucrose in homogenization buffer lacking NP40, cyclohexamide in heparin at 28,000 RPM for eight hours at 4°C in a Beckman SW28 rotor. The supernatant was aspirated off and the pellets were resuspended in 4 ml extraction buffer comprising 50 mM tris-HCl pH 9, 1 mM EDTA, 150 mM NaOAc and 1% sodium dodecylsulfate (SDS). Proteinase K was added to 250 µg/ml and the mixture was incubated at 37°C for 30 minutes. The Polysomal RNA was phenol-chloroform extracted twice and precipitated at -20°C with the addition of 1/10 volume 3M NaOAc and 2 volumes 95% ethanol.

## Example II
### Preparation of Polyadenylated mRNA from Polysomal RNA

Poly(A)+ mRNA was obtained by hybridization to oligo d(T)- cellulose columns (2X). The column was equilibrated with binding buffer having a composition of 10mM tris-HCl pH 7.4, 1mM EDTA, 0.1% SDS, 400 mM NaCl and 10% glycerol, and the whole polysomal RNA preparation of Example I was cycled through the column 5 times. Unbound RNA was eluted with several column volumes of binding buffer. Elution buffer having a composition of 10mM tris-HCl, pH 7.4, 1mM EDTA and 0.1% SDS, was used to wash bound poly(A)+ RNA from the columns and it was precipitated as before. The following describes the process of oligo dT column chromatography in greater detail.

Stock Solutions and Materials:

Oligo dT binding buffer (1X): 10 mM Tris.HCl, pH 7.6, 1mM EDTA, 0.4 M NaCl, (0.1% SDS)

Oligo dT binding buffer (2X): 10 mM Tris.HCl, pH 7.4, 1mM EDTA, 0.8 M NaCl, (0.1% SDS)

Oligo dT elution buffer: 10 mM Tris.HCl, pH 7.4, 1mM EDTA, (0.1% SDS).

Procedure:

(1) Pellets of precipitated RNA were thoroughly drained of EtOH, resuspended in oligo dT elution buffer, and absorbances at 260 nm and 280 nm were determined; $A_{260/280}$ should be about 2, and an $A_{260}$ of 1.0 corresponds to an RNA concentration of 40 µg/ml.

(2) The RNA solution was then adjusted to 5-10 mg/ml with the elution buffer and heated for 5 minutes at 65°C, quick-cooled and mixed with an equal volume of the 2X oligo dT binding buffer.

(3) The RNA solution was chromatographed on an oligo dT column (about 2 g in a column of 0.9 cm diameter) which had previously been washed with 5 column volumes of 1X oligo dT binding buffer; the column was eluted at a rate of about 10-15 ml/hr.

(4) The column effluent was recycled once, then retained as "poly A" RNA and stored under ethanol.

(5) The oligo dT column was then washed with about 5-8 column volumes of 1X oligo dT binding buffer, and then RNA was eluted with about two to three column volumes of elution buffer and collected in fractions of about 25-35 drops with the aid of a Gilson fraction collector; RNA generally eluted between fractions 16 and 25.

(6) Absorbances of the fractions were determined and the UV-absorbing material pooled to produce the 1X oligo dT purified "poly A+ RNA".

(7) At this point, the pooled RNA fractions were either rechromatographed on an oligo dT cellulose column to further purify the poly A+ RNA (step 7a) or precipitated with ethanol and stored until used or further purified on oligo dT cellulose (step 7b):

(a) Rechromatography:

after step (6), the volume of the pooled fractions was determined, the solution was heated to 65°C for 5 minutes, quick-cooled and diluted with an equal volume of 2X oligo dT binding buffer and passed over the same oligo dT column which had been thoroughly washed with several column volumes of elution buffer and several volumes of binding buffer; RNA was then processed as in steps 3, 4, 5, and 6, and then precipitated by addition of sodium acetate to 0.3 $\underline{M}$ and 2.5 volumes of 95% ethanol (-20°C to -80°C). The resulting precipitate was designated "2X purified oligo dT poly A+ RNA".

(b) Ethanol precipitation followed by rechromatography: if after step (6), a stopping was desired, the RNA solution was made 0.3M in sodium acetate and 2.5 volumes of 95% ethanol were added; after at least 2 hours at -20°C, RNA was pelleted by centrifugation at 10000xg for 15 min, washed 2X with 70% EtOH and drained; RNA was the processed, starting with step (1) but the initial concentration of poly A+ RNA should be about 1 mg/ml or less.

(8) After use, the oligo dT column was cleaned by passing 2-3 column volumes of 0.1 N KOH or NaOH through it. The column was then washed with 5-7 column volumes of oligo dT binding buffer containing 0.02% sodium azide, and stored at room temperature.

The integrity of the poly(A)+ RNA was checked by translation in a rabbit reticulocyte cell free system supplemented with $^{35}$S-methionine commercially available from Bethesda Research Laboratories, Rockville, Maryland, U.S.A. The products of these translations were visualized by sodium dedecyl sulfate polyacrylamide gel electrophoresis and fluorography.

Example III
Preparation of Enriched OGH mRNA for
Double-Stranded cDNA Synthesis

Ovine growth hormone mRNA was separated from the poly(A)+ RNA of Example II as described Nilson, et al., Nucleic Acids Research, 8:1561-1573 (1980) except that (1) the RNA was heated to 80°C for 2 minutes, then quick cooled on ice prior to loading on the 5%-20% sucrose gradient; (2) centrifugation was at 30,000 RPM for 20 hours at 22°C in a Beckman SW40 rotor. The RNA present in ethanol precipitated 0.5 ml fractions was translated in the rabbit reticulocyte system and the products visualized by gel

1.7

electrophoresis and fluorography.  Fractions containing OGH mRNA were pooled.

## Example IV
## Synthesis of Double-Stranded cDNA

Stock Solutions and Materials for First Strand cDNA Synthesis

0.5 M Tris HCl, pH 8.3

1.4 M KCl

0.25 M $MgCl_2$

0.05 M dATP, pH 7.0

0.05 M dGTP, pH 7.0

0.05 M dCTP, pH 7.0

0.05 M dTTP, pH 7.0

$[\alpha-^{32}P]dCTP$, 400 Ci/mmol, 1 mCi/ml (Amersham)

0.01 M dithiothreitol (DTT)

Oligo (dT)12-18' 250 µg/ml (Collaborative Research)

Actinomycin D, 500 µg/ml (Calbiochem)

0.2 M disodium ethylenediaminetetraacetate (EDTA), pH 8.0.

Avian myeoblastosis virus reverse transcriptase, approximately 10,000 units/ml (obtained from Research Resources Branch, Viral Oncology Program, National Cancer Institute)

All buffers and salt solutions were autoclaved.  The other solutions were prepared with sterile glass-distilled water and were stored in sterile containers.  All stock solutions were stored frozen.

Procedure:

As a template for cDNA synthesis, mRNA isolated as in Example III was employed.  In order to follow the synthesis, a radioactive marker such as $[\alpha-^{32}P]dCTP$ was used.  The radioactive compound was dried by lyophilization, blowing off the solvent with a stream of nitrogen, or by drying in a Savant Speed Vac Concentrator.  For each ug of mRNA, 5 uCi of $[\alpha-^{32}P]dCTP$ at a specific activity of 400 ci/mmol were used. The dried material was dissolved in a 2X reaction mixture consisting of 0.1 M Tris HCl, pH 8.3, 140 mM KCl, 20 mM $MgCl_2$, 1 mM dATP, 1mM dCTP, 1mM dGTP, 1mM TTP, and 0.4 mM DTT. This solution was kept on ice. To this solution were added mRNA (50 μg/ml, final concentration), oligo(dT)$_{12-18}$ (25 μg/ml), actinomycin D (40 μg/ml), AMV reverse transcriptase (800 units/ml), and enough water to dilute the mix 2 to 1. After 5 min on ice, the reaction mixture was incubated at 46° for 10 min. Following the incubation, EDTA was added to a final concentration of 25 mM. The solution was extracted one time with an equal volume of phenol:chloroform (1/1; v/v) and the aqueous phase was chromatographed on a column of Sephadex G-100 (0.7 x 20 cm) equilibrated with 10 mM Tris.HCl, pH 8.0, 1 mM EDTA, 0.1 M NaCl. The cDNA in the excluded volume was precipitated by addition of 0.1 volume of 3 M NaOAc and 2 volumes of 95% ethanol (-20°C to -80°C). In order to remove the mRNA moiety the pelleted complex was dissolved in 300 μl 0.1 M NaOH and incubated at 70°C for 20 minutes. The solution was cooled on ice and neutralized with 30 μl of 1 N HCl. The cDNA was re-precipitated as described above.

Stock Solutions and Materials
for Second Strand cDNA Synthesis

0.5 M potassium phosphate, pH 7.4

0.25 M $MgCl_2$

0.1 M DTT

0.05 M dATP, pH 7.0

0.05 M dGTP, pH 7.0

0.05 M dCTP, pH 7.0

0.05 M TTP, pH 7.0

$[\alpha-^{3}H]dCTP$, 22 Ci/mmol, 1 mCi/ml (Amersham)

E.coli DNA polymerase I (Klenow fragment), approximately 1000 units/ml (Boehringer-Mannheim) S1 nuclease buffer: 0.167 M sodium acetate, pH 4.5; 5 mM $ZnCl_2$

Procedure:

It was not necessary to use a radioactive label in the second strand, since the first strand was labelled. A 2X reaction mixture consisting of 0.2 M potassium phosphate, pH 7.4, 20 mM MgCl$_2$, 2 mM DTT, 0.4 mM each of dATP, dGTP, dCTP, and TTP were prepared and kept on ice. To this mixture was added an aqueous solution of cDNA containing the Klenow fragment of E.coli DNA polymerase I (100 units/ml. final concentration), and water was added to dilute the reaction mixture to 1. The solution was incubated a minimum of 2 hours at 15°C. It was convenient to allow this reaction to incubate overnight. After the incubation, EDTA was added to 25 mM, the solution was extracted once with an equal volume of phenol:chloroform (1/1; v/v), and the aqueous phase was chromatographed on a 0.7 x 20 cm column of Sephadex G-100 equilibrated with 10 mM Tris-HCl, pH 8.0, 1 mM EDTA, and 0.1 M NaCl. The DNA in the excluded fractions was precipitated with ethanol as described above.

At this point, the ds cDNA contained a hairpin loop. The single-stranded loop was removed by digestion with Aspergillus oryzae S1 nuclease. The ds cDNA was dissolved in water and 0.20 volume of 5x S1 buffer was added. An appropriate amount of S1 nuclease was added and the solution was incubated 20 min at 37°. The amount of enzyme added was determined empirically for each enzyme preparation, since the activity varied from one preparation to another. This was done by measuring the decrease in TCA-precipitable counts from the ds cDNA. The S1-digested cDNA was extracted once with phenol:chloroform and the aqueous phase was precipitated with ethanol as described above.

## Example V

### Homopolymeric Tailing and Sizing of ds cDNA

Double-stranded cDNA was inserted into a cloning vector by adding single-stranded homopolymeric tails to the 3'-OH termini of the ds cDNA and complementary homopolymeric tails to the 3'-OH termini of the vector. The two tailed molecules were then annealed.

Stock Solutions and Materials:

1.4 M potassium cacodylate, 0.3 M Tris, pH 7.6 (pH becomes 7.2 when diluted 1:10)

15 mM $CoCl_2$

10 mM DTT

4 mM dCTP, pH 7.0

Procedure:

Sizing of the ds cDNA allows an accurate estimation of the concentration of 3'-termini. Addition of dCMP residues to the 3' ends was followed by incorporation of $[^3H]$dCTP into acid-precipitable material. Twenty-five μCi of $[^3H]$dCTP were used for each ug of mRNA used for the original cDNA synthesis. The radioactive compound was dried and redissolved in the reaction mixture. Double-stranded cDNA was dissolved in appropriate amounts of stock solutions to give final concentrations as follows: 0.14 M potassium cacodylate, 0.03 M Tris, pH 7.2; 1 mM DTT; 0.1 mM $[^3H]$dCTP (1 Ci/mmol); 1.5 mM $CoCl_2$; and 2 x $10^{-8}$ M 3'-termini. The solution without $CoCl_2$ was warmed to 37°; the $CoCl_2$ was then added. Terminal deoxynucleotidyl transferase (purified by the method of Chane, L.M.S. et al. J. Biol. Chem. 246, 909 [1971]) was added to a final concentration of 100 units/ml. The reaction was allowed to proceed at 37°C for 5 min. At this time, a sample was taken to measure incorporaton of $[^3H]$dCMP into acid-precipitable material. If tails of sufficient length were not added, the reaction was continued by placing the solution at 37°C again for the desired length of time. The best transformation efficiencies were obtained with homopolymer tails of about 10-20 dCMP residues. When tails of the desired length were generated, EDTA was added (10 mM, final concentration).

The solution was extracted once with phenol:chloroform (1/1; v/v) and the aqueous phase was precipitated with ethanol.

Tailed double-stranded cDNA was sized on neutral sucrose gradients as outlined by Norgard, et al. (J. Biol. Chem., 255:7665-7672 [1980]). The ds cDNA was dissolved in distilled $H_2O$ and layered on chilled 12 ml 5%-25% sucrose gradient (50mM tris HCl pH7.5, 1mM EDTA). The gradients were spun at 39,000 RPM, for 17.5 hr. at 5°C in a Beckman SW40 rotor. A second gradient containing 10 mg of Taq 1 endonuclease digested plasmid pBR322 DNA for size standards was run simultaneously. The gradients were fractionated and analyzed as follows: (a) Taq 1 digested pBR322 gradient - To each 0.5 ml fraction were added 5 mg tRNA (E.coli), one-tenth volume 3M NaOAc, 2 volumes 95% ethanol and precipitated @ -20°C to -80°C. The DNA is pelleted and dried in vacuo. The pellet is resuspended in sample buffer (0.09M tris pH 8.5, 0.09M Boric Acid, 40mM EDTA, 5% glycerol, 0.025% xylene cyanol, 0.025% Bromphenyl blue) and electrophoresed on an 8% acrylamide (TBE) horizontal slab gel. The ethidium bromide stained gel was photographed and lanes containing DNA greater than 600 base pairs were noted. (b) ds cDNA Gradient - 100 µl of each 1 ml fraction was added to 13 ml of scintillate for counting. Then to each fraction was added 10 mg tRNA (E.coli), 1/10 volume 3M NaOAc and precipitated with 2.5 volumes 95% ETOH at -80°C. Only fractions containing ds cDNA longer than 600 base pairs (as indicated by the Taq 1 digested pBR322 gel information) were used in subsequent steps.

## Example VI

### Preparation of Hybrid Plasmids

Oligo d(G) tails were added to PstI - linearized pBR322 by the procedure described in Example V. The oligo(dG) - tailed pBR322 DNA and sized oligo (dC) tailed ds cDNA were diluted to equimolar concentrations in 10 mM

22

tris-HCl pH 7.5, 100mM NaCl, 2.5mM Na$_2$ EDTA. The DNAs were annealed at 70°C for 10' and then cooled slowly overnight to room temperature.

## Example VII
### Transformation

Recombinant plasmids were introduced into E.coli HB101 according to the method of Lederberg and Cohen (J. Bact., 119:1072 [1074]). Transformants were grown on standard LB (L-broth)-tetracycline (15 mg/ml) plates at 30°C for 2 days. Colonies were picked to LB-amp (50 mg/ml) plates to determine phenotype. Only ampicillin sensitive colonies were selected for further analysis.

## Example VIII
### Preparation of a Nick Translated Probe

A nick translated bovine growth hormone fragment (prepared as described in U.S. patent application serial number 442,584 filed November 18, 1982) was employed as a screening probe for identifying possible OGH clones from among the transformants produced (Example VII). An internal Pst-1 DNA fragment containing approximately half the coding region for bovine growth hormone was isolated from pGX1201 using the acrylamide gel method of Hansen, J.N., Analytical Biochemistry, 116:146-151 (1981). The fragment was resuspended in distilled H$_2$O and P$^{32}$-labelled according to the nick translation procedure of Rigby, P.W.S., et al., Journal of Molecular Biology, 113:237-251 (1977). The nick ranslated fragment was denatured by incubation at 100°C for five minutes just prior to its addition to the hybridization mix (Example IX).

## Example IX
### Identifying Recombinant Plasmids

Transformants were prepared for colony hybridization according to a modification of the procedure originally described by Grunstein, M. et al. PNAS, 72:3961-3965 (1975). The DNA bound filters were hybridized in the presence of dextran sulfate and formamide as suggested by Wahl, G., et al., PNAS, 76:3683-3687 (1979). The filters were pre-hybridized in 6xSSC (20x = 3M NACl; 0.3M Na₃ utrate); 1x Denhardt's solution (10x = 0.2% BSA; 0.2% PVP-40; 0.2% ficoll); 0.1% SDS in a glass jar with gentle swirling at 42°C for 60'. A second pre-hybridization followed in 8 mls/filter at 50% formamide, 5x SSC, 5x Denhardt's solution, 50 mM NaPO₄ pH 6.5, 1% glycine, 195 ug/ml denatured salmon sperm DNA for 2 hours at 40°C with gentle swirling. The nick-translated P[32]-labelled BGH DNA probe described in Example VIII was added to the hybridization mix (10[6]cpm/filter) containing 50% formamide, 5xSSC, 1x Denhardt's solution, 20mM NaPO₄ pH 6.5, 10% dextran sulfate, 100 μg/ml denatured salmon sperm DNA and incubated with the filters at 42°C-18 hours with gentle swirling. The filters were washed 3x10' in 2x SSC, 0.1% SDS at room temperature and 2x10' in 0.1x SSC, 0.1% SDS at 50°C. Positives were visualized by exposing the blotted filters to Kodak XR-5 film overnight at 80°C with an intensifying screen.

## Example X
### Plasmid Isolation and Analysis

Plasmid DNA was extracted from each positive clone and electrophoresed on 1% agarose gels made in accordance with the method of Helling, et al., J. of Virology, 14:1235 (1974). Plasmid DNA containing inserts larger than 500 base pairs were selected for further analysis by restriction endonuclease digestion with Pst I and Ava I. Several such plasmids were identified. The plasmid containing the largest insert was sequenced by the method of Sanger (supra) and was determined to contain the full length pre-OGH gene. This pre-OGH gene was excised and isolated by gel electrophoresis, again as described by Sanger (supra) and was designated and deposited as NRRL No. B-15060.

1.    A cloned ovine growth hormone gene.

2.    A cloned ovine growth hormone gene as claimed in claim 1, comprising a cloned pre-ovine growth hormone gene.

3.    An intron-free cloned ovine growth hormone gene.

4.    An intron-free cloned ovine growth hormone gene as claimed in claim 3 comprising a cloned pre-ovine growth hormone gene.

5.    The ovine growth hormone gene comprising the following deoxyribonucleotide sequence:

```
127
Ala Phe Pro Ala Met Ser Leu Ser Gly Leu Phe Ala Asn Ala Val
GCX TTY CCX GCX ATG QRS YTZ QRS GGX YTZ TTY GCX AAY GCX GTX
172
Leu Arg Ala Gln His Leu His Gln Leu Ala Ala Asp Thr Phe Lyr
YTZ LGN GCX CAM CAY YTZ CAY CAM YTZ GCX GCX GAY ACX TTY AAM
217
Glu Phe Glu Arg Thr Tyr Ile Pro Glu Gly Gln Arg Tyr Ser Ile
GAM TTY GAM LGN ACX TAY ATH CCX GAM GGX CAM LGN TAY QRS ATH
262
Gln Asn Thr Gln Val Ala Phe Cys Phe Ser Glu Thr Ile Pro Ala
CAM AAY ACX CAM GTX GCX TTY TGY TTY QRS GAM ACX ATH CCX GCX
307
Pro Thr Gly Lys Asn Glu Ala Gln Gln Lys Ser Asp Leu Glu Leu
CCX ACX GGX AAM AAY GAM GCX CAM CAM AAM QRS GAY YTZ GAM YTZ
352
Leu Arg Ile Ser Leu Leu Leu Ile Gln Ser Trp Leu Gly Pro Leu
YTZ LGN ATH QRS YTZ YTZ YTZ ATH CAM QRS TGG YTZ GGX CCX YTZ
397
Gln Phe Leu Ser Arg Val Phe Thr Asn Ser Leu Val Phe Gly Thr
CAM TTY YTZ QRS LGN GTX TTY ACX AAY QRS YTZ GTX TTY GGX ACX
442
Ser Asp Arg Val Tyr Glu Lys Leu Lys Asp Leu Glu Glu Gly Ile
QRS GAY LGN GTX TAY GAM AAM YTZ AAM GAY YTZ GAM GAM GGX ATH
487
Leu Ala Leu Met Arg Glu Leu Glu Asp Val Thr Pro Arg Ala Gly
YTZ GCX YTZ ATG LGN GAM YTZ GAM GAY GTX ACX CCX LGN GCX GGX
532
Gln Ile Leu Lys Gln Thr Tyr Asp Lys Phe Asp Thr Asn Met Arg
CAM ATH YTZ AAM CAM ACX TAY GAY AAM TTY GAY ACX AAY ATG LGN
577
Ser Asp Asp Ala Leu Leu Lys Asn Tyr Gly Leu Leu Ser Cys Phe
QRS GAY GAY GCX YTZ YTZ AAM AAY TAY GGX YTZ YTZ QRS TGY TTY
622
```

```
Arg Lys Asp Leu His Lys Thr Glu Thr Tyr Leu Arg Val Met Lys
LGN AAM GAY YTZ CAY AAM ACX GAM ACX TAY YTZ LGN GTX ATG AAM
667
Cys Arg Arg Phe Gly Glu Ala Ser Cys Ala Phe
TGY LGN LGN TTY GGX GAM GCX QRS TGY GCX TTY
```

wherein, the 5' to 3' strand, beginning with the amine terminus and the amino acids for which each triplet codes are shown, and wherein within each triplet,

A is deoxyadenyl

T is thymidyl

G is deoxyguanyl

C is deoxycytosyl

X is A, T, C or G

Y is T or C

When Y is C, Z is A, T, C or G

When Y is T, Z is A or G

H is A, T or C

Q is T or A

When Q is T, R is C and S is A, T, C or G

When Q is A, R is G and S is T or C

M is A or G

L is A or C

When L is A, N is A or G

When L is C, N is A, T, C or G

Gly is glycine

Ala is alanine

Val is valine

Leu is leucine

Ile is isoleucine

Ser is serine

Thr is threonine

Phe is phenylalanine

Tyr is tyrosine

Trp is tryptophan

Cys is cysteine

Met is methionine

Asp is aspartic acid

Glu is gluatmic acid

Lys is lysine

Arg is arginine

His is histidine

Pro is proline

Gln is gluatmine

Asn is asparagine

6. The ovine pre-growth hormone gene of claim 2, comprising the following deoxyribonucleotide sequence:

```
49
Met Met Ala Ala Gly Pro Arg Thr Ser Leu Leu Leu Ala Phe Thr
ATG ATG GCX GCX GGX CCX LGN ACX QRS YTZ YTZ YTZ GCX TTY ACX
94
Leu Leu Cys Leu Pro Trp Thr Gln Val Val Gly Ala Phe Pro Ala
YTZ YTZ TGY YTZ CCX TGG ACX CAM GTX GTX GGX GCX TTY CCX GCX
139
Met Ser Leu Ser Gly Leu Phe Ala Asn Ala Val Leu Arg Ala Gln
ATG QRS YTZ QRS GGX YTZ TTY GCX AAY GCX GTX YTZ LGN GCX CAM
184
His Leu His Gln Leu Ala Ala Asp Thr Phe Lys Glu Phe Glu Arg
CAY YTZ CAY CAM YTZ GCX GCX GAY ACX TTY AAM GAM TTY GAM LGN
229
Thr Tyr Ile Pro Glu Gly Gln Arg Tyr Ser Ile Gln Asn Thr Gln
ACX TAY ATH CCX GAM GGX CAM LGN TAY QRS ATH CAM AAY ACX CAM
274
Val Ala Phe Cys Phe Ser Glu Thr Ile Pro Ala Pro Thr Gly Lys
GTX GCX TTY TGY TTY QRS GAM ACX ATH CCX GCX CCX ACX GGX AAM
319
Asn Glu Ala Gln Gln Lys Ser Asp Leu Glu Leu Leu Arg Ile Ser
AAY GAM GCX CAM CAM AAM QRS GAY YTZ GAM YTZ YTZ LGN ATH QRS
364
Leu Leu Leu Ile Gln Ser Trp Leu Gly Pro Leu Gln Phe Leu Ser
YTZ YTZ YTZ ATH CAM QRS TGG YTZ GGX CCX YTZ CAM TTY YTZ QRS
409
Arg Val Phe Thr Asn Ser Leu Val Phe Gly Thr Ser Asp Arg Val
LGN GTX TTY ACX AAY QRS YTZ GTX TTY GGX ACX QRS GAY LGN GTX
454
Tyr Glu Lys Leu Lys Asp Leu Glu Glu Gly Ile Leu Ala Leu Met
TAY GAM AAM YTZ AAM GAY YTZ GAM GAM GGX ATH YTZ GCX YTZ ATG
499
Arg Glu Leu Glu Asp Val Thr Pro Arg Ala Gly Gln Ile Leu Lys
LGN GAM YTZ GAM GAY GTX ACX CCX LGN GCX GGX CAM ATH YTZ AAM
544
Gln Thr Tyr Asp Lys Phe Asp Thr Asn Met Arg Ser Asp Asp Ala
CAM ACX TAY GAY AAM TTY GAY ACX AAY ATG LGN QRS GAY GAY GCX
589
```

```
Leu Leu Lys Asn Tyr Gly Leu Leu Ser Cys Phe Arg Lys Asp Leu
YTZ YTZ AAM AAY TAY GGX YTZ YTZ QRS TGY TTY LGN AAM GAY YTZ
634
His Lys Thr Glu Thr Tyr Leu Arg Val Met Lys Cys Arg Arg Phe
CAY AAM ACX GAM ACX TAY YTZ LGN GTX ATG AAM TGY LGN LGN TTY
679
Gly Glu Ala Ser Cys Ala Phe
GGX GAM GCX QRS TGY GCX TTY
```

wherein the 5' to 3' strand, beginning with the amine terminus, and the amino acids for which each triplet codes are shown, and wherein the abbreviations are defined as in claim 3.

7. The ovine growth hormone gene of claim 1, comprising the following deoxyribonucleotide sequence: wherein the 5' to 3' strand, beginning with the amine terminus, and the amino acids for which each triplet codes are shown, and wherein the abbreviations are defined as in claim 5:

```
127
Ala Phe Pro Ala Met Ser Leu Ser Gly Le. Phe Ala Asn Ala Val
GCC TTC CCA GCC ATG TCC TTG TCC GGC CTG TTT GCC AAC GCT GTG
172
Leu Arg Ala Gln His Leu His Gln Leu Ala Ala Asp Thr Phe Lys
CTG CGG GCT CAG CAC CTG CAT CAA CTG GC'' GCT GAC ACC TTC AAA
217
Glu Phe Glu Arg Thr Tyr Ile Pro Glu Gl, Gln Arg Tyr Ser Ile
GAG TTT GAG CGC ACC TAC ATC CCG GAG GG' CAG AGA TAC TCC ATC
262
Gln Asn Thr Gln Val Ala Phe Cys Phe Se: Glu Thr Ile Pro Ala
CAG AAC ACC CAA GTT GCC TTC TGC TTC TC' GAA ACC ATC CCG GCC
307
Pro Thr Gly Lys Asn Glu Ala Gln Gln Ly: Ser Asp Leu Glu Leu
CCC ACG GGC AAG AAT GAG GCC CAG CAG AA. TCA GAC TTG GAG CTG
352
Leu Arg Ile Ser Leu Leu Leu Ile Gln Ser Trp Leu Gly Pro Leu
CTT CGC ATC TCA CTG CTC CTT ATC TCG TCG 'GG CTT GGG CCC CTG
397
Gln Phe Leu Ser Arg Val Phe Thr Asn Ser Leu Val Phe Gly Thr
CAG TTC CTC AGT AGA GTC TTC ACC AAC AGC ( TG GTG TTT GGC ACC
442
Ser Asp Arg Val Tyr Glu Lys Leu Lys Asp Leu Glu Glu Gly Ile
TCG GAC CGT GTC TAT GAG AAG CTG AAG GAC ( TA GAG GAA GGC ATC
487
Leu Ala Leu Met Arg Glu Leu Glu Asp Val Thr Pro Arg Ala Gly
CTA GCC CTG ATG CGG GAG CTG GAA GAT GTT ; CC CCC CGG GCT GGG
```

```
532
Gln Ile Leu Lys Gln Thr Tyr Asp Lys Phe Asp Thr Asn Met Arg
CAG ATC CTC AAG CAG ACC TAT GAC AAA TTT GAC ACA AAC ATG CGC
577
Ser Asp Asp Ala Leu Leu Lys Asn Tyr Gly Leu Leu Ser Cys Phe
AGT GAT GAT GCG CTG CTC AAG AAC TAC GGT CTG CTC TCC TGC TTC
622
Arg Lys Asp Leu His Lys Thr Glu Thr Tyr Leu Arg Val Met Lys
CGG AAG GAC CTG CAC AAG ACG GAG ACG TAC CTG AGG GTC ATG AAG
667
Cys Arg Arg Phe Gly Glu Ala Ser Cys Ala Phe
TGC CGC CGC TTC GGG GAG GCC AGC TGT GCC TTC
```

wherein the 5' to 3' strand, beginning with the amine terminus, and the amino acids for which each triplet codes are shown, and wherein the abbreviations are defined as in claim 5.

8. The ovine pre-growth hormone gene of claim 2, comprising the followng deoxyribonucleotide sequence:

```
49
Met Met Ala Ala Gly Pro Arg Thr Ser Leu Leu Leu Ala Phe Thr
ATG ATG GCT GCA GGC CCC CGG ACC TCC CTG CTC CTG GCT TTC ACC
94
Leu Leu Cys Leu Pro Trp Thr Gln Val Val Gly Ala Phe Pro Ala
CTG CTC TGC CTG CCC TGG ACT CAG GTG GTG GGC GCC TTC CCA GCC
139
Met Ser Leu Ser Gly Leu Phe Ala Asn Ala Val Leu Arg Ala Gln
ATG TCC TTG TCC GGC CTG TTT GCC AAC GCT GTG CTC CGG GCT CAG
184
His Leu His Gln Leu Ala Ala Asp Thr Phe Lys Glu Phe Glu Arg
CAC CTG CAT CAA CTG GCT GCT GAC ACC TTC AAA GAG TTT GAG CGC
229
Thr Tyr Ile Pro Glu Gly Gln Arg Tyr Ser Ile Gln Asn Thr Gln
ACC TAC ATC CCG GAG GGA CAG AGA TAC TCC ATC CAG AAC ACC CAA
274
Val Ala Phe Cys Phe Ser Glu Thr Ile Pro Ala Pro Thr Gly Lys
GTT GCC TTC TGC TTC TCT GAA ACC ATC CCG GCC CCC ACG GGC AAG
319
Asn Glu Ala Gln Gln Lys Ser Asp Leu Glu Leu Leu Arg Ile Ser
AAT GAG GCC CAG CAG AAA TCA GAC TTG GAG CTG CTT CGC ATC TCA
364
Leu Leu Leu Ile Gln Ser Trp Leu Gly Pro Leu Gln Phe Leu Ser
CTG CTC CTT ATC CAG TCG TGG CTT GGG CCC CTG CAG TTC CTC AGT
409
Arg Val Phe Thr Asn Ser Leu Val Phe Gly Thr Ser Asp Arg Val
AGA GTC TTC ACC AAC AGC CTG GTG TTT GGC ACC TCG GAC CGT GTC
454
Tyr Glu Lys Leu Lys Asp Leu Glu Glu Gly Ile Leu Ala Leu Met
TAT GAG AAG CTG AAG GAC CTA GAG GAA GGC ATC CTA GCC CTG ATG
499
```

```
Arg Glu Leu Glu Asp Val Thr Pro Arg Ala Gly Gln Ile Leu Lys
CGG GAG CTG GAA GAT GTT ACC CCC CGG GCT GGG CAG ATC CTC AAG
544
Gln Thr Tyr Asp Lys Phe Asp Thr Asn Met Arg Ser Asp Asp Ala
CAG ACC TAT GAC AAA TTT GAC ACA AAC ATG CGC AGT GAT GAT GCG
589
Leu Leu Lys Asn Tyr Gly Leu Leu Ser Cys Phe Arg Lys Asp Leu
CTG CTC AAG AAC TAC GGT CTG CTC TCC TGC TTC CGG AAG GAC CTG
634
His Lys Thr Glu Thr Tyr Leu Arg Val Met Lys Cys Arg Arg Phe
CAC AAG ACG GAG ACG TAC CTG AGG GTC ATG AAG TGT CGC CGC TTC
679
Gly Glu Ala Ser Cys Ala Phe
GGG GAG GCC AGC TGT GCC TTC
```

wherein the 5' to 3' strand, beginning with the amine terminus, and the amino acids for which each triplet codes are shown, and wherein the abbreviations are defined as in

claim 5.

9.  The ovine pre-growth hormone gene of claim 2, comprising the following deoxyribonucleotide sequence:

```
1
GGG GGG GGG GGG GAG ACG ACT CAG GGT CCT GCT GAC AGC TCA CCA GCT
49
Met Met Ala Ala Gly Pro Arg Thr Ser Leu Leu Leu Ala Phe Thr
ATG ATG GCT GCA GGC CCC CGG ACC TCC CTG CTC CTG GCT TTC ACC
94
Leu Leu Cys Leu Pro Trp Thr Gln Val Val Gly Ala Phe Pro Ala
CTG CTC TGC CTG CCC TGG ACT CAG GTG GTG GGC GCC TTC CCA GCC
139
Met Ser Leu Ser Gly Leu Phe Ala Asn Ala Val Leu Arg Ala Gln
ATG TCC TTG TCC GGC CTG TTT GCC AAC GCT GTG CTC CGG GCT CAG
184
His Leu His Gln Leu Ala Ala Asp Thr Phe Lys Glu Phe Glu Arg
CAC CTG CAT CAA CTG GCT GCT GAC ACC TTC AAA GAG TTT GAG CGC
229
Thr Tyr Ile Pro Glu Gly Gln Arg Tyr Ser Ile Gln Asn Thr Gln
ACC TAC ATC CCG GAG GGA CAG AGA TAC TCC ATC CAG AAC ACC CAA
274
Val Ala Phe Cys Phe Ser Glu Thr Ile Pro Ala Pro Thr Gly Lys
GTT GCC TTC TGC TTC TCT GAA ACC ATC CCG GCC CCC ACG GGC AAG
319
Asn Glu Ala Gln Gln Lys Ser Asp Leu Glu Leu Leu Arg Ile Ser
AAT GAG GCC CAG CAG AAA TCA GAC TTG GAG CTG CTT CGC ATC TCA
364
Leu Leu Leu Ile Gln Ser Trp Leu Gly Pro Leu Gln Phe Leu Ser
CTG CTC CTT ATC CAG TCG TGG CTT GGG CCC CTG CAG TTC CTC AGT
409
Arg Val Phe Thr Asn Ser Leu Val Phe Gly Thr Ser Asp Arg Val
AGA GTC TTC ACC AAC AGC CTG GTG TTT GGC ACC TCG GAC CGT GTC
```

```
454
Tyr Glu Lys Leu Lys Asp Leu Glu Glu Gly Ile Leu Ala Leu Met
TAT GAG AAG CTG AAG GAC CTA GAG GAA GGC ATC CTA GCC CTG ATG
499
Arg Glu Leu Glu Asp Val Thr Pro Arg Ala Gly Gln Ile Leu Lys
CGG GAG CTG GAA GAT GTT ACC CCC CGG GCT GGG CAG ATC CTC AAG
544
Gln Thr Tyr Asp Lys Phe Asp Thr Asn Met Arg Ser Asp Asp Ala
CAG ACC TAT GAC AAA TTT GAC ACA AAC ATG CGC AGT GAT GAT GCG
589
Leu Leu Lys Asn Tyr Gly Leu Leu Ser Cys Phe Arg Lys Asp Leu
CTG CTC AAG AAC TAC GGT CTG CTC TCC TGC TTC CGG AAG GAC CTG
634
His Lys Thr Glu Thr Tyr Leu Arg Val Met Lys Cys Arg Arg Phe
CAC AAG ACG GAG ACG TAC CTG AGG GTC ATG AAG TGT CGC CGC TTC
679
Gly Glu Ala Ser Cys Ala Phe
GGG GAG GCC AGC TGT GCC TTC TAG TTG CCA GCC ATC TGT TGT TAC

CCC TCC CCC GTG CCT TCC TAG ACC CTG GAA GGT GCC ACT CCA GTG

CCC ACT GTC CTT TCC TAA TAA AAT GAG GAA ATT GCA TCG C
```

wherein the 5' to 3' strand, beginning with the amine terminus, and the amino acids for which each triplet codes are shown, and wherein the abbreviations are defined as in claim 5.

10 . A plasmid having the capability of replication in a procaryotic organism, comprising a deoxynucleotide sequence coding for ovine growth hormone.

11 . A plasmid having the capability of replication in a procaryotic organism, comprising the ovine growth hormone gene as claimed in any of claims 1 to 9.

12 . The plasmid of claim 11, wherein said procaryotic organisms is of the genus _Escherichia_.

13 . A microorganism transformed by the plasmid of claim 11.

14 . The microorganism of claim 13, of the genus _Escherichia_.

15 . The microorganism of claim 14, of the species _coli_.

16 . A method for producing ovine pre-growth hormone, which comprises cultivating on an aqueous nutrient medium containing assimilable sources of carbon, nitrogen and essential minerals and growth factors, under ovine pre-growth hormone-producing conditions, a procaryotic organism transformed by a plasmid capable of replicating in said organism and having a deoxynucleotide sequence coding for ovine pre-growth hormone; and recovering ovine pre-growth hormone so produced.

17.    A method for producing ovine growth hormone, which comprises cultivating on an aqueous nutrient medium containing assimilable sources of carbon, nitrogen and essential minerals and growth factors, under ovine growth hormone-producing conditions, a procaryotic organism transformed by a plasmid capable of replicating in said organism and having a deoxynucleotide sequence coding for ovine growth hormone; and recovering ovine growth hormone so produced.

18.    The method of claim 16 or claim 17 wherein the procaryotic organisms is Escherichia coli.

19 .    The method of claim 18, wherein said procaryotic organism is substantially similar to NRRL No. B-15060.

20.    A biologically pure culture of E. coli strain NRRL No. B-15060.

# FIG.1

| | | | |
|---|---|---|---|
| 0 - PST I | 60 - PST I | 398 - PST I | 808 - PST I |

European Patent Office

**EUROPEAN SEARCH REPORT**

EP 84302700.4

| Category | Citation of document with indication, where appropriate, of relevant passages | Relevant to claim | CLASSIFICATION OF THE APPLICATION (Int. Cl. ³) |
|---|---|---|---|
| A | EP - A2 - 0 047 600 (UNIVERSITY OF CALIFORNIA)<br>* Claims 1-5,7,8,12 *<br>-- | 1-18 | C 12 N 15/00<br>C 12 P 21/00<br>C 12 P 19/34//<br>C 12 R. 1/19 |
| A | EP - A1 - 0 020 147 (UNIVERSITY OF CALIFORNIA)<br>* Claims 1-5,7,10,12 *<br>-- | 1-18 | |
| A | GB - A - 2 073 245 (YEDA)<br>-- | | |
| A,D | SCIENCE, vol. 205, no. 4406, August 1979, Washington, D.C.<br>J.A. MARTIAL et al. "Human Growth Hormone: Complementary DNA Cloning and Expression in Bacteria"<br>pages 602-606<br>-- | | |
| A | NATURE, vol. 281, October 1979, London<br>D.V. GOEDDEL et al. "Direct expression in Escherichia coli of a DNA sequence coding for human growth hormone"<br>pages 544-548<br>---- | | |

## DOCUMENTS CONSIDERED TO BE RELEVANT

TECHNICAL FIELDS SEARCHED (Int. Cl. ³)

C 12 N
C 12 P

The present search report has been drawn up for all claims

| Place of search | Date of completion of the search | Examiner |
|---|---|---|
| VIENNA | 26-07-1984 | FARNIOK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82